# EUROPEAN PATENT APPLICATION

(11) **EP 0 543 222 A1**
(43) Date of publication of application: **26.05.1993**
(21) Application number: 92118867.8
(22) Date of filing: 04.11.1992
(51) Int. Cl.: C12Q 1/68, C12Q 1/70

(54) **Method for detecting nucleic acids and relative diagnostic kit**

(30) Priority: 15.11.1991 IT MI913048
(71) Applicant: ALFA BIOTECH S.p.A., I-00040 Pomezia (Roma) (IT)
(72) Inventor: Curcio, Stefano, I-00040 Pomezia, Roma (IT); Cellentani, Gabriella, I-00040 Pomezia, Roma (IT); Fochesato, Natalia, I-00040 Pomezia, Roma (IT); Padula, Cristiano, I-00040 Pomezia, Roma (IT)
(74) Representative: Minoja, Fabrizio

(57) **Abstract**

Method for detecting specific sequences of a given nucleic acid consisting of:
A. the formation of an amplified product of the extension of a first pair of primer oligonucleotides complementary to the nucleic acid to be detected, at least one of which is labelled with a specific ligand;
B. the formation of a hybrid complex between the amplified extension product and a labelled DNA probe;
C. the immobilization of the hybrid complex on a solid phase sensitized with a receptor capable of stably fixing the ligand mentioned in point A;
D. the detection of the probe-amplified product complex adhering to the solid phase with immunoenzymatic type methods;
characterized by the fact that the labelled DNA probe is obtained by means of an amplification procedure with a second pair of primer oligonucleotides inside the oligonucleotides of the first pair on the sequence of the extension product.

## Description

This invention concerns a method for detecting specific sequences of a given nucleic acid in a sample. The method of the invention may be essentially used in any sector in which nucleic acids are involved: diagnostics, biomedical research, forensic medicine, industrial biopharmacology, zootechny, agricultural-food sector and so on.

The main diagnostic applications include genetic diseases, infectious diseases and the study of tumours.

Nucleic acids are known to represent the maximum expression of individual specificity and the procedures that permit their analysis and identification have aroused enormous scientific interest for some time and are becoming increasingly more important.

In the case of infectious diseases, for example, the possibility of recognizing in a biological sample the presence of characteristic DNA or RNA sequences of a given micro-organism has a greater diagnostic value than that of any other method used for the direct or indirect (serological) detection of other components of the micro-organism (proteins, polysaccharides, etc.).

In the case of genetic diseases, it is possible to make a diagnosis on the basis of the presence of certain variations in the sequences that make up the genes, regardless of the expression of the genes themselves.

DNA has a highly stable double helix structure, but the two helixes can be separated (denaturation) with physical (heat) or chemical means (pH, formaldehyde).

If denaturation is obtained with heat, when the temperature is lowered again the two helixes return to their original nature (renaturation).

If the renaturation procedure occurs in the presence of a DNA (or an RNA) with a sequence that is complementary to one of the two helixes, a hybrid is formed between the so-called target DNA (or RNA) and the homologous sequence used as a probe, thus giving rise to the term hybridization.

Under certain working conditions the formation of a hybrid may be used to reveal the presence of a given target sequence: if the hybrids that are formed adhere to a solid phase which permits their separation from the rest of the unpaired molecules and if the probe has been appropriately labelled, the presence of a hybridization signal in correspondence with the solid phase will indicate the presence of the target sequence.

All the aspects described above, from the hybridization techniques to the probe labelling, from the use of the solid phases to the detection systems, have been the object of detailed studies performed by numerous authors and there are many patents concerning the method to carry out the various passages mentioned.

The denaturation of nucleic acids, necessary if the target DNA (or RNA) and/or probe have a double helix, is described for example in Gergen J.P. et al. (Nucleic Acid Research. 1980, 7:2115-2136).

Hybridization is a per se known technique and is described for example in EP-A-82102673.9 dated 13 October 1982.

The reference method, based on the transfer of DNA from agar gel to nitrocellulose filters or similar and the subsequent hybridization, is described by Southern (1975) and is named after him.

The probe may consist of a single or double helix DNA or RNA, extracted from living organisms or from tissues, or inserted into different cells, usually bacteria, according to a procedure defined as cloning. These techniques are described for example by Krist et al. (Proc. Natl. Acad. Sci. USA, 1981. 78:2772).

The labelling system generally consists of the insertion of a nucleotide containing a radioactive atom into the probe sequence, so that its presence can be detected with autoradiography systems or by scintigraphy. Thus, for example, EP-A-68206 describes a method with the relative kit for the detection of the Hepatitis B virus through hybridization of nucleic acids. The probe is radioactively labelled and its presence is detected by scintigraphy. One of the limitations of these systems is the need to handle radioactive substances, with the consequent problems linked to the short half-life of these substances and their potential danger.

Various non-radioactive labelling methods have been proposed to overcome the disadvantages of the radio-isotope systems. EP-A-82321824.9 describes some methods for the covalent binding to DNA of small molecules, including biotin, that are able to form stable complexes with some polypeptides. In the case of biotin, high affinity peptides can be used, such as avidin or streptavidin, or anti-biotin immunoglobulins. Both avidin (or streptavidin) and the immunoglobulins can be recognized by a specific antibody to which an enzyme is bound (peroxidase or alkaline phosphatase). The enzyme is able to catalyse the reaction that converts a colourless medium into a coloured substance which is directly visible or detectable with photometric methods.

Streptavidin or anti-biotin immunoglobulin can also be bound to the enzyme directly, thus reducing the number of steps necessary.

The presence of biotin on the DNA molecule does not create appreciable interference with the hybridization phenomena, and biotin-labelled probes can therefore be used to obtain hybrids which can be detected with one of the methods described above.

A different approach involves the chemical modification of DNA so that it can be recognized by a specific antibody; DNA in itself is not in fact a good antigen, but once it has undergone certain chemical modifications it may become so.

Numerous articles describe the existence of antibodies for modified DNA (Munns and Liszewski, in "Antibodies Specific for Modified Nucleosides: An Immunochemical Approach for the Isolation and Characterization of Nucleic Acid").

One DNA alkylation technique is described by Rajewsky et al. (Immunological detection, etc., Carcinogen Fundamental Mechanism and Environmental Effect, 207-218, 1980, Reidel Publishing Co.).

Briecos et al. (Biochemistry, 1978, 17:1986) describe the use of specific antibodies for 0-6-Methylguanine that selectively bind to alkylated DNA.

The possibility of obtaining a DNA sulfonation is discussed by Poverenny et al. (Molecular Immunology, 1979, 16:313, Pergamon Press).

EP-A-128018 proposes some types of DNA chemical modification, including alkylation, sulfonation and others, as labelling methods to prepare probes for use in hybridization reactions, making use of specific antibodies for the detection of modified DNA.

The technique used to produce the antibodies is described by Muller R. et al. (10 IARC Scientific Publ., 39:436).

The enzymes bound to the antibodies may be peroxidases, beta-galactosidases, hyaluronidases, alkaline phosphatases or others.

The numerous non-radioactive labelling systems that have been proposed, although they resolve the problems linked to the use of radioactive isotopes, have not yet improved the sensitivity of the tests based on the use of nucleic acid probes. On the contrary, the sensitivity of non-radioactive probes is generally lower than that of the isotopic systems, which from this point of view still represent the reference model, with a sensitivity threshold of around 0.1 picogram of DNA.

This threshold is often insufficient to provide useful information for clinical purposes, as occurs for example with viral infections.

A completely new method of dealing with the problem is envisaged with the introduction of a new technique, called Polymerase Chain Reaction or PCR, described in US-A-4,683,195 and US-A-4,683,202, that consists of in vitro enzymatic replication (amplification) of a given DNA sequence caused by a thermostable polymerase DNA enzyme.

The DNA tract to be amplified is detected by the enzyme thanks to the inclusion, in the reaction mixture, of two small single-helix DNA molecules, obtained by synthesis (primer oligonucleotides). Due to their complementary nature, the ends of these molecules bind to the region to be amplified.

This is a universal technique, since it can be applied to any type of DNA for which at least part of the nucleotidic sequence is known. The two primers are designed and synthesized on the basis of this sequence. The first one must bind to one helix of the target DNA, the second to the complementary helix. The distance that separates the two regions recognized by the primers, (varying from tens to 2-3 thousand nucleotides), defines the length of the amplified fragment.

If a sequence of RNA (e.g. of a retrovirus) is to be amplified, amplification must be preceded by a passage in which the RNA is transformed into DNA by the reverse transcriptase RNA enzyme.

Once the amplification procedure has been achieved, there is the problem of the method to be used to detect the presence of the amplified products.

The "traditional" procedure, usually considered as a reference, involves the following steps: an aliquot of the mixture that has undergone the amplification procedure is subjected to electrophoretic migration in agar gel, to allow the separation of DNA fragments according to their length and secondary structure.

After migration, the gel is immersed in a solution containing ethidium bromide, a substance that binds to DNA and emits a red-orange fluorescence under ultraviolet light.

In this way, using a DNA with a known-length as a reference, it is possible to establish whether amplification has produced a visible amount of DNA and whether the amplified fragment is approximately the expected length.

The intensity of the fluorescent lane, compared with that of the reference DNA, allows a semi-quantitative estimate of the amplification product.

This method can provide an indication of the results of the amplification procedure, but it does not make it possible to establish whether the sequence of the amplified DNA corresponds exactly to the expected one.

Despite a careful choice of the oligonucleotides (primers) there is, in fact, always the possibility that the amplification procedure may cause the production of one or more aspecific lanes. One of these lanes may even be apparently similar in length to what is expected, thus giving a misleading indication.

Confirmation by hybridization with a specific probe is always necessary in order to be certain that the amplified fragment has a sequence corresponding to what is desired, and further steps must therefore be carried out.

The reference method considered (Southern) requires the transfer of DNA fragments from gel to a nylon or nitro-cellulose filter and then hybridization with a specific probe. In this case, thanks to amplification, a great degree of sensitivity is not required and non-radioactive probes may therefore be used.

As an alternative to gel-electrophoresis and the Southern method, the "dot-blot" method can be used (see Maniatis et al. Molecular Cloning. A Laboratory Manual. Cold Spring Harbor Laboratory, 1982), making a drop of amplified DNA adhere directly to a filter and then proceeding with hybridization of the filter with a probe.

The main disadvantage of these otherwise very reliable methods is the time required to perform them, which involves numerous laborious and delicate steps.

In particular, the use of filters as solid supports and the need to carry out a whole series of operations on the spot has so far discouraged the use of these methods in ordinary laboratories.

In view of the great potential offered by the DNA amplification technique, it is desirable to have a method for the detection of the amplification products that is simple and rapid, accurate, sensitive and reliable, prepared in a kit form for immediate use in any laboratory.

With this objective EP-A-370694 and EP-A-0417842 describe PCR amplification product detection techniques that basically consist of the following stages:
A. The formation of an amplification product of the extension of a first pair of primer oligonucleotides complementary to the nucleic acid that is to be detected, at least one of which being labelled with a specific ligand;
B. The formation of a hybrid complex between the amplified extension product and a labelled DNA probe;
C. The immobilization of the hybrid complex on a solid phase sensitized with a receptor capable of stably fixing the ligand mentioned in point A;
D. The detection of the probe-amplified complex adhering to the solid phase with immunoenzymatic type methods.

Although on paper these systems appear to offer advantages with respect to the above-mentioned Southern and "dot-blot" techniques, they are limited as oligonucleotides, which are not able to offer a high selectivity and are unsuitable for certain types of labelling_{,} are used as probes.

The present invention overcomes the drawbacks of the previous technique, providing a method for detecting specific sequences of a given nucleic acid including:
A. The formation of an amplification product of the extension of a first pair of primer oligonucleotides complementary to the nucleic acid that must be detected, at least one of which being labelled with a specific ligand;
B. The formation of a hybrid complex between the amplified extension product and a labelled DNA probe;
C. The immobilization of the hybrid complex on a solid phase sensitized with a receptor capable of stably fixing the ligand mentioned in point A;
D. The detection of the probe-amplified product complex adhering to the solid phase with immunoenzymatic type methods;
characterized by the fact that the labelled DNA probe is obtained by means of an amplification procedure with a second pair of primer oligonucleotides inside the oligonucleotides of the first pair on the sequence of the extension product.

The method of the invention envisages in particular the use of a DNA probe obtained by means of an amplification procedure with a second pair of primer oligonucleotides ("B" pair), chosen so that, when they come into contact with the same sequence recognized by the "A" primers, they give rise to an amplification product that is different from that obtained with the "A" pair due to the total or partial absence of the extremities corresponding to the "A" primers and to their complementary tract.

In other words, the primer oligonucleotides of the "B" pair can be defined as being inside the oligonucleotides of the "A" pair on the target DNA sequence.

The amplification product obtained in this way is inserted in an appropriate vector and replicated inside a bacterial host according to the procedures known as cloning.

The oligonucleotides of the "B" pair can be designed to have sequences, close to the 5' end, that can be recognized by the restriction enzymes, to be used in the cloning process before the vector is inserted.

The entire vector-insert complex is appropriately labelled and used as a probe.

The present invention provides a rapid method for identifying a given nucleic acid in a sample even if only minimal traces are present.

The use of a probe with the above-described features ensures enormous specificity, since the complementary nature extends for a sequence corresponding almost entirely to the amplification product. The hybridization conditions can therefore be made extremely "stringent" thus eliminating practically every possibility of aspecific pairing of the probe.

Since a bond is used between a receptor adhering to the solid phase and a ligand fixed to one or both of the primers as a system for immobilizing the amplification product, the probe must not have sequences that are complementary to the primers used for amplification, since they would bind to these and thus to the solid phase even in the absence of the amplification product, as the primers are a fixed component of the reaction.

To overcome this aspect, the present invention proposes to use for the production of the probe an appropriate amplification procedure with a second pair of primers, chosen so as to amplify a slightly smaller fragment than the one produced by the first pair of primers, to avoid having in common with the latter sequences which are long enough to give rise to a complementary bond.

The DNA labelling systems that involve an "immunogenic" chemical modification are preferable since they have excellent sensitivity, specificity and are practical, but they are not generally very efficient in labelling small fragments, such as oligonucleotides or even the amplification products, since the latter are generally designed within a length range of between 100 and 1000 base pairs.

Using a probe consisting of an amplified fragment together with the vector used for its cloning, it is possible to obtain a marked increase in labelling efficiency, since the total dimensions are well above 1,000 base pairs, and are generally between 2,000 and 10,000 base pairs, depending on the vector used and the length of the amplified fragment.

By adding "tails", containing sequences that can be recognized by restriction enzymes, to the second pair of primers, the cloning operations are accelerated and considerably simplified, also providing a system for the "selection" of the correct fragments to be inserted in the vector.

The present invention is particularly effective when used in combination with a DNA amplification procedure, carried out using a mixture consisting of the following:
a) A part of the sample in which the target DNA (or RNA) is to be detected, appropriately treated.
b) A reaction buffer.
c) Triphosphate nucleotides (dATP, dGTP, dCTP, dTTP).
d) Two oligonucleotides (primers), specific for the DNA to be amplified, at least one of which is modified to permit its capture by a specific agent adhering to a solid phase.
e) A DNA polymerizing agent.

The elements listed above can be supplied in kit form, ready for immediate use.

The nucleic acid to be amplified may be of various origins. It may be DNA or RNA of whatever origin: plasmidic, viral, bacterial, vegetable, animal or human. It may come from various tissues, including blood, mononucleate cells of peripheral blood, urine, secretions and any other possible source.

The nucleic acids may be isolated with any one of the known techniques, provided this does not lead to a degradation of the DNA (or RNA).

The techniques for the extraction and purification of the nucleic acids in the sample may in many cases be replaced by a simple treatment consisting of bringing the sample to boiling point for a few minutes, to release the DNA (or RNA) and at the same time disactivating the enzymes and foreign substances that may interfere with amplification.

If an RNA sequence is to be amplified, the sample must be subjected to the action of the reverse transcriptase enzyme, which synthesizes a DNA copy using the RNA in the sample as the pattern. The subsequent steps are the same.

The reaction sample and the triphosphate deoxyribonucleotide mixture are in accordance with what is described in patent US-A-4,683,202.

The primer sequence is appropriately selected on the basis of the DNA sequence to be amplified, according to the criteria generally adopted for this type of choice. To date numerous primer sequences are already known and have been shown to be effective in the amplification of the most diverse target DNAs.

The primers generally have a single helix, with a length of between 15 and 60 nucleotides, preferably between 20 and 30 nucleotides, and can be obtained with one of the numerous known techniques, such as for example the isolation of fragment mixtures resulting from the action of restriction enzymes, or, preferably, can be produced synthetically using an automatic oligonucleotide synthesizer.

The present invention requires that at least one of the primers has a modification that allows its recognition by a specific agent.

The modification generally consists of the addition of one or more ligands that can be biotin or its derivatives, avidin or streptavidin, sulfhydrylic groups, lectins, carbohydrates, proteins, haptens, drugs, antibodies or other compounds. All these ligands possess reactive groups that allow them to bind to the primers, directly or through spacer molecules.

Each ligand has the property of forming a stable complex with given molecules, called receptors, by means of a highly specific interaction.

Once the ligand has been selected, the choice of the receptor follows as a consequence. So, for example, if the ligand is biotin, the receptor will be avidin, streptavidin or an analogous derivative; if the ligand is an antigen, the receptor will be the specific antibody and so on.

Since the primer oligonucleotides are incorporated in their extension product (or amplification product), it is possible to obtain a system for the capture of the amplified products using a receptor adhering to a solid phase. The use of ligands and receptors for immobilizing the primers and the amplification products is described in EP-A-370694.

In a preferred version of the present invention two oligonucleotides are used as primers, at least one of which is biotinylated at the 5' end using one of the standard methods, such as for example the procedure described by Kempe et al., (Nucleic Acid Res. 13, 45-47, 1985).

Biotin does not noticeably interfere with the activity of the primers for which it is more important to have the 3' end free. It is also preferable to use a spacer molecule between the biotin and the DNA to minimize the reciprocal interferences.

The polymerizing agent should preferably be a thermostable DNA-polymerase enzyme, of natural origin or produced by cloning, starting from some thermophilic bacterial species such as Thermus thermophilus and Thermus aquaticus. These and other polymerizing agents, as well as the methods for obtaining them, are disclosed in USA-A-4,683,202 and in EP-A-258,017.

The amplification procedure, described in detail in USA-A-4,683,202, takes place through a repeated series of thermal cycles that generally involve a denaturation stage (at 90-95°C), a primer pairing stage (at 35-75°C) and an extension stage of the paired primers (at 70-75°C). The thermal cycles may be automated with the use of an appropriate apparatus.

The amplification protocol can be adapted each time to the specific circumstances and may even undergo marked variations, maintaining however its fundamental characteristic of the production of a large number of copies of the DNA sequence that is to be detected.

At the end of the thermal cycles, an aliquot of the amplification mixture, appropriately diluted, is mixed with a labelled DNA probe (for the characteristics of the probe see below) and incubated at a constant temperature for a relatively short time (hybridization in the liquid stage is very rapid), with the addition of a suitable hybridization buffer.

The probe is able to bind to the primer extension products forming hybrid molecules, that derive from the pairing of an amplified helix with a probe helix.

At the end of the hybridization step, the mixture is incubated in contact with a solid phase sensitized with a receptor appropriately chosen on the basis of the ligand used for the primers.

The solid phase may consist of membranes, microspheres, wells or any other means that offers a surface suitable for sensitization with the receptor, before being placed in contact with the reaction mixture containing the ligand.

In a preferred version, in which one or both of the primers are labelled with biotin at the 5' end, the solid phase consists of plastic wells (polystyrene or similar) like those commonly used for immunoenzymatic tests on plates and is sensitized with streptavidin according to a standard procedure.

At the end of incubation, some washing cycles are used to remove all the components that have not been captured on the solid phase.

To detect the probe adhering to the solid phase, an agent that can bind specifically to the labelled probe is used, preferably an antibody, which can in turn be detected with an immunoenzymatic method.

In a preferred version, the probe is labelled with the addition of sulfhydrylic groups (see below) and the probe-amplified product complex captured on the solid phase is detected by an anti-DNA-sulfhydrylate antibody, directly conjugated with an enzyme or recognized by a second antibody conjugated with an enzyme, that catalyses a colourimetric reaction.

For the first antibody, preference is given to a monoclonal anti-DNA-sulfhydrylate antibody, obtained by Nur et al. (Ann.Biol. Clin. 1989; 47:601) with the method described by Kohler and Milstein (Nature 1975; 256:495) and thus of murin origin.

For the second antibody, an anti-immunoglobulin antiserum from mouse conjugated with an enzyme is used. The enzyme may be alkaline phosphatase, peroxidase, or any other suitable one. Preferably a goat anti mouse IgG (Fc) antiserum is used, conjugated with peroxidase.

The product of the colourimetric reaction catalysed by the enzyme starting from an appropriate medium may give rise to a coloured precipitate on the solid phase (if a chromogen that forms insoluble precipitates is used), or may remain in solution and give this a certain degree of colour (using soluble chromogens). In a preferred version, in which the enzyme is peroxidase and the solid phase consists of plastic wells, a mixture of hydrogen peroxide and orthophenylenediamine is used as the chromogen medium.

When a soluble chromogen is used, the intensity of the staining obtained is proportional to the degree of positiveness of the sample tested and, if the reaction takes place in a well, can be measured with an appropriate plate reader (commonly used in immunoenzymatic methods) thus obtaining a result expressed in numerical form.

In accordance with the invention, the DNA probe is obtained by means of an amplification procedure with a second pair of primer oligonucleotides ("B" pair), the "A" pair being the two oligonucleotides considered up until now for the sample amplification procedure.

The oligonucleotides of the "B" pair recognize the same extension product of the "A" primers, but they bind themselves to it in a more "inner" position, so as to completely or partially exclude the extremes of the sequence corresponding to the "A" primers and to their complementary tract.

One or both of the oligonucleotides of the "B" pair can be designed to have an additional sequence at the 5' end, even not complementary to the tract to be amplified. These sequences, preferably of a length between 1 and 10 nucleotides, are chosen so that they can be recognized, once they have become part of the extension product, by specific restriction enzymes for each sequence.

This makes it possible to create extension products with extremities that are compatible with any cloning site.

The DNA sequence used as a pattern by the "B" pair oligonucleotides may have various origins. It may be contained in a positive sample, it may be part of a purified DNA mixture or it may have a synthetic or other origin. Preference is given to the "A" primer extension product, appropriately diluted.

The standard procedure is used for oligonucleotide amplification with the "B" pair.

The "B" pair primer amplification product is roughly purified and, if necessary, treated with the appropriate restriction enzymes if the relative recognition sequences have been added.

Insertion in an appropriate vector and subsequent replication inside a bacterial host is performed according to the normal procedures known as cloning.

The complex consisting of the vector and the insert, once replicated in the desired quantity, is purified and then labelled appropriately.

The labelling method used is not critical for the purpose of this invention, which is however particularly effective in combination with a labelling of the entire insert+vector complex by DNA sulfhydrylation (or sulfonation), as described by Sverdlov et al. (Biochim. Biophys. Acta 1974, 340:153-165).

In the presence of sodium bisulfate and methoxyamine, substances that can stably bind -SH groups to cytosine residues, DNA undergoes a chemical modification. The cytosinic residue is thus transformed into N-4-methoxy-5,6-dihydrocytosine-6 sulfonate. The sulfonation involves on average 10-15% of the cytosinic residues, equivalent to 2-3% of all the nucleotidic residues of a DNA with a mean G-C composition of 47%.

The sulfonation procedure used is the same as that described by Sverdlov (op. cit.).

The diagnostic kit based on the present invention can be divided into two sections, the first of which is specific for each nucleic acid sequence to be detected, while the second is of universal use.

The two sections can be packed together or separately. The first section is designed for sample amplification and hybridization with the probe and contains as its critical component the DNA probe for the specific amplified product that the user wishes to detect, a probe characterized by the fact that it is prepared with the previously described method.

The first section of the kit also contains two primer oligonucleotides, the extension product of which is recognized by the above-mentioned probe since at least one of the said primers is labelled with a ligand, preferably biotin.

The first section of the kit also provides the buffers necessary for the hybridization in the liquid stage.

Several probes with their respective primer pairs may be provided in the same package.

Optional kit components are the buffer for DNA amplification, the triphosphate deoxyribonucleotides dATP, dGTP, dDTP, dTTP, a polymerizing agent and one or more samples suitable for use as positive and/or negative controls.

The kit may contain a system for the preliminary treatment of the sample, consisting of filtering elements or other equipment, buffer solutions, solvents, lysis agents, surface-active agents, deproteinizers and anything else that may in turn be considered useful on the basis of the sample characteristics and the nucleic acid that must be treated.

The invention kits that have RNA as their target can contain the solutions and reagents necessary for the reverse transcription of RNA to DNA.

The second section of the kit contains a receptor for the specific ligand, this receptor being stably fixed to a solid phase. In a preferred version in which the ligand is biotin, the receptor is avidin, streptavidin or a similar derivate.

The solid phase preferably consists of plastic wells (polystyrene or similar) of the type commonly used for immunoenzymatic tests.

The second section of the kit also contains an antibody, that recognizes the labelled DNA, directly conjugated with an enzyme or recognized by a second antibody, also provided with the kit, conjugated with an enzyme.

In a preferred version, in which the probe is labelled by sulfonation, the first antibody is a monoclonal anti-DNA-sulfonate while the second is an anti mouse IgG antiserum labelled with an enzyme, preferably peroxidase.

The second section of the kit may also contain buffer solutions, enzymatic substrates, chromogens, washing buffers, diluents, blocking solutions and all the other components and materials used for immunoenzymatic detection.

All the reagents in both sections of the kit can be packed individually or pre-mixed, in a concentrated or freeze-dried form or ready for use.

Various accessories can also be provided together with the kit such as test-tubes, pipettes, clamps, containers, instructions and anything else that may be of use for the application of this invention.

### EXAMPLE

In the following example the primer sequences are reported according to the convention that envisages the use of the four initials to indicate the nucleotides (A = adenine, G = guanine, C = cytosine, T = thymine). All the sequences are written in the order going from the 5' end to the 3' end.

The tris (hydroxymethyl)aminomethane-hydrochloride component is indicated as Tris-HCl.

### DETECTION OF CYTOMEGALOVIRUS DNA IN BIOLOGICAL SAMPLES

This example demonstrates the practical application of the present invention for the detection in biological samples of a nucleic acid sequence that is specific for the human Cytomegalovirus.

The biological samples used for the detection of the Cytomegalovirus (CMV) are urine and serum.

Urine is diluted 1:5 in Tris-HCl 0.5 M pH 8.3, boiled for 20 minutes at 100°C and subsequently placed in ice. Ten µl of the solution thus treated is used for amplification.

Serum is diluted 1:3 in Tris-HCl 0.5 M pH 8.3, boiled for 20 minutes at 100°C, placed in ice and centrifuged for 5 minutes at 13000 rpm. Ten µl of the supernatant is used for amplification.

The primers used are described in literature (Cranage MP et al. EMBO, 1986, 5:3057) and have the following nucleotidic sequence:

1: GGAATCCAGGATCTGGTGCCTG
2: GTAGTGTTGTAGATAGTCTC

These primers, which will be referred to as CMV1 and CMV2, recognize a sequence belonging to the glycoprotein B gene and give rise to an extension product with a length of 221 base pairs.

The primers were synthesized with an automatic device (Applied Biosystem) and subsequently modified with biotinylation of the 5' end.

The amplification mixture has a volume of 100 µl and contains:
a) Tris-HCl pH 8.3 (100 mM)
b) Magnesium chloride (1.5 mM)
c) Potassium chloride (25 mM
d) Gelatin (100 µg/ml)
e) 20 picomoles of each biotinylated primer (CMV1 and CMV2)
f) 25 micromoles of each triphosphate deoxynucleotide (dATP, dGTP, dCTP, dTTP)
g) 10 µl of sample, treated in the above-mentioned manner
h) DNA polymerases of Thermus aquaticus (1.5 units), commercially available.

The reaction mixture was covered with 75 µl of mineral oil to prevent evaporation and then subjected to 43 amplification thermal cycles in an automatic device according to the following scheme:
- Denaturation: 95°C for 60 seconds
- Primer pairing: 52°C for 90 seconds
- Extension: 72°C for 60 seconds.

The extension stage was prolonged by 10 seconds at each successive cycle.

The probe for the amplified extension product of the Cytomegalovirus was obtained as follows: on the basis of the sequence (known) of the extension product of the biotinylated CMV1 and CMV2 primers, two new primers were designed, indicated as CMV3 and CMV4, which give rise to a smaller extension product, in which the sequences relative to the CMV1 and CMV2 primers are missing.

The sequence of the CMV3 primer was designed to contain a recognition site for the restriction enzyme Eco RI in the 5' proximal portion, whereas the CMV4 primer sequence contains a recognition site for the Pst I enzyme, again in the 5' proximal portion.

The sequence of the CMV3 and CMV4 primers is given below, with the restriction sites underlined:

CMV3: GGGAATTCTGCGTTAACCTGTGTATCGT
CMV4: GGGCTGCAGTTGGCTCTATGACTGACGG

Amplification with the CMV3 and CMV4 primers is performed with a mixture containing:
a) Tris-HCl pH 8.3 10 mM
b) Magnesium chloride (1.5 mM)
c) Potassium chloride (50 mM)
d) Gelatin (100 µg/ml)
e) 50 picomoles of each primer (CMV3 and CMV4)
f) 25 micromoles of each triphosphate deoxynucleotide (dATP, dGTP, dCTP, dTTP)
g) 6 ng of DNA extracted from human fibroblasts infected with CMV, strain AD 169, according to a standard procedure as regards the infection, the cultivation of the infected cells and the extraction/purification of the DNA with proteinase K, phenol/chloroform and ethanol (see for example Kimpton C.P., J. Vir. Meth., 1989, 24:335).
h) 1.5 units DNA polymerase of Thermus aquaticus, commercially available.

The reaction mixture was covered with 75 µl of mineral oil to prevent evaporation and then subjected to 30 amplification thermal cycles in an automatic device according to the following scheme:
- Denaturation: 95°C for 60 seconds
- Primer pairing: 52°C for 90 seconds
- Extension: 72°C for 60 seconds.

The extension stage was prolonged by 10 seconds at each successive cycle.

The extension product of the primers CMV3 and CMV4 was then subjected to the action of the restriction enzymes Eco Ri and Pst I and incubated, in the presence of the enzyme DNA ligase and ATP, with a vector, represented by the phage M13 mp18 (Boehringer Mannheim), previously subjected to digestion with Eco RI and Pst I.

The product of ligation was inserted in Escherichia coli cells, strain 7118, rendered permeable (competent) with a treatment based on cold calcium chloride.

The transformed cells were distributed on plates containing LB, in the presence of fresh Escherichia coli cells, and incubated overnight at 37°C.

The growth of the phage was revealed by the appearance of lysis plaques on the layer of bacterial growth.

A series of mini-cultures were set up starting from single plaques and after growing overnight at 37°C the plasmidic DNA was extracted with the alkaline lysis method and digested with the Eco RI and Pst I enzymes.

The presence of the insert was checked on 2% agar gel in a Tris-borate-EDTA buffer and the supranatant of the corresponding culture was used to set up a liquid culture in 500 ml of LB to prepare the DNA for use as a probe.

The plasmidic DNA obtained from the bacterial pellet was purified on ion-exchange columns (Quiagen^{©} -Diagen), precipitated in ethanol, dried, resuspended in distilled water and quantified.

For DNA labelling by sulfonation, a commercial kit (Chemiprobe, FMC) was used according to the instructions provided by the manufacturer, bringing the probe to a final dilution of 50 ng/µl.

The hybridization reaction between the amplification product and the probe took place as follows:
a) One aliquot of the amplified product was diluted 1:5 with sterile distilled water.
b) 5 µl of diluted amplified product was mixed with 10 µl (500 ng) of DNA probe prepared with the method described above and with 10 µl of sterile bidistilled water.
c) The mixture was incubated for 10 minutes at 100°C
d) 25 µl of a hybridization solution was added containing:
   - SSC 12x
   - sodium dodecylsulfate (SDS) 0.2%
   - Denhart's solution (Ficoll, Polyvinylpyrrolidone, Bovine serum albumin) 10x
e) The hybridization mixture is kept at 65°C for one hour.

The hybrid complex formed from the biotinylated amplification and the labelled probe with sulfhydrylic groups is then detected with an immunoenzymatic method in wells.

For the solid phase, strips of 8 polystyrene wells were used (NUNC maxisorp).

Streptavidin (Chemicon), at an initial concentration of 1 mg/ml, was diluted 1:100 in a buffer Na₂CO₃/NaHCO₃ 0.005 N pH 9.6.

The solutions containing streptavidin was distributed in the wells (125 µl/well) and incubated overnight at 4°C.

At the end of the incubation period two washings were made at room temperature with 300 µl/well of a solution containing Tris-HCl 0.005 M, NaCl 0.9% and Tween 20 0.005%.

For well saturation 125µl of a solution containing Tris-HCl 0.05 M, NaCl 0.9% (pH 7.75) and BSA 0.5% was used and left to incubate overnight at 4°C.

The strips that are not used immediately can be dried and stored at 4°C for several months.

At the end of the steps described above, the hybridization mixture was diluted in PBS buffer 0.006 M pH 7.3.

100 µl of this dilution was incubated in a well sensitized with streptavidin (see above).

Incubation was carried out at 37°C for 90 minutes.

At the end of the incubation period, three 1-minute washings were performed at room temperature with 300 µl/well of a solution of PBS 0.004 M and Tween 20 0.01%.

After the washings, 100 µl of monoclonal mouse anti-DNA sulfhydrylate antibody (Chemiprobe® FMC) diluted 1:2000 in PBS 0.004 M, Tween 20 0.01% BSA 0.5% were distributed in the well and left to incubate for 60 minutes at 37°C.

At the end of the incubation period, three 1-minute washings were made at room temperature with 300 µl/well of a solution of PBS 0.004 M and Tween 20 0.01%.

After the washings 100 µl of a goat anti mouse IgG (Fc) antiserum, conjugated with peroxidase (Biospacific), diluted 1:200 in PBS 0.004 M, Tween 20 0.01% were distributed in the well and incubated for 30 minutes at 37°C.

At the end of the incubation period, three 1-minute washings were made at room temperature with 300 µl/well of a solution of PBS 0.004 M and Tween 20 0.001%.

One hundred µl of a medium/chromogen solution for peroxidase (prepared diluting orthophenylendiamine and hydrogen peroxide in a citrated-phosphate buffer at pH 5.0) were then placed in the well and left to incubate for 10 minutes at 37°C.

The enzymatic reaction is blocked by adding 100 µl of sulphuric acid 2 M to the well.

The intensity of the stain obtained is read with a spectrophotometer (reader for immunoenzymatic plates) at 492 nm and converted into a numerical value.

The positivity of each sample can be assessed by comparison with the values provided in the same analysis session by the negative (distilled water and/or negative samples) and positive controls (DNA extracted from fibroblasts infected with CMV and/or positive samples).

## Claims

1. Method for detecting specific sequences of a given nucleic acid consisting of:
A. the formation of an amplified product of the extension of a first pair of primer oligonucleotides complementary to the nucleic acid to be detected, at least one of which is labelled with a specific ligand;
B. the formation of a hybrid complex between the amplified extension product and a labelled DNA probe;
C. the immobilization of the hybrid complex on a solid phase sensitized with a receptor capable of stably fixing the ligand mentioned in point A;
D. the detection of the probe-amplified product complex adhering to the solid phase with immunoenzymatic type methods;
characterized by the fact that the labelled DNA probe is obtained by means of an amplification procedure with a second pair of primer oligonucleotides inside the oligonucleotides of the first pair on the sequence of the extension product.

2. Method as in claim 1 characterized by the fact that at least one of the primers of the first pair is biotinylated at the 5' end and that the hybrid between the biotinylated extension product and the labelled probe is immobilized on a solid support sensitized with streptavidin.

3. Method as in claim 1 or 2, characterized by the fact that the solid phase consists of plastic wells, of the type commonly used for immunoenzymatic tests on plates.

4. Method as in any one of the aforementioned claims characterized by the fact that the probe is labelled with sulfhydrylic groups and that the probe-amplified hybrid is detected with immunoenzymatic type methods using a monoclonal anti-DNA-sulfhydrylate antibody.

5. Method as in any one of the aforementioned claims characterized by the fact that one or both of the oligonucleotides of the second pair of primers has an additional sequence in 5' that can be recognized by a restriction enzyme.

6. Method as in any one of the aforementioned claims characterized by the fact that the labelled probe includes a portion which acts as a vector to permit reproduction inside a host cell.

7. Method as in any one of the aforementioned claims in which the amplified product is Cytomegalovirus DNA.

8. Diagnostic kit containing:
a) a first section that consists of:
- a pair of primer oligonucleotides, at least one of which being labelled with a ligand;
- a labelled probe designed for the specific amplified product, obtained by means of an amplification procedure with a second pair of primer oligonucleotides inside the oligonucleotides of the first pair on the sequences of the extension product;
- buffers suitable for hybridization in the liquid stage;
b) a second section including:
- a receptor fixed on a solid phase for the ligand conjugated with one or both of the primer nucleotides;
- one or more agents able to specifically recognize the probe labelling.
